Europäisches Patentamt

European Patent Office

Office européen des brevets

(19)

(11) **EP 1 571 448 A1**

(12) **EUROPEAN PATENT APPLICATION**
published in accordance with Art. 158(3) EPC

(43) Date of publication:
**07.09.2005 Bulletin 2005/36**

(51) Int Cl.⁷: **G01N 33/68**, G01N 33/483,
C12N 15/09, C12N 1/21,
C12N 5/10, C07K 14/435

(21) Application number: **03778776.9**

(22) Date of filing: **10.12.2003**

(86) International application number:
**PCT/JP2003/015790**

(87) International publication number:
**WO 2004/053499 (24.06.2004 Gazette 2004/26)**

(84) Designated Contracting States:
**AT BE BG CH CY CZ DE DK EE ES FI FR GB GR
HU IE IT LI LU MC NL PT RO SE SI SK TR**

(30) Priority: **10.12.2002 JP 2002357768**

(71) Applicants:
• **RIKEN
Saitama 351-0198 (JP)**
• **Japan Science and Technology Agency
Saitama 332-0012 (JP)**

(72) Inventors:
• **MIYAWAKI, Atsushi c/o RIKEN
Wako-shi, Saitama 351-0198 (JP)**
• **NAGAI, Kenji c/o RIKEN
Wako-shi, Saitama 351-0198 (JP)**

(74) Representative:
**Sternagel, Fleischer, Godemeyer & Partner
Patentanwälte,
An den Gärten 7
51491 Overath (DE)**

(54) **FLUORESCENT INDICATOR USING FRET**

(57)     It is an object of the present invention to provide a fluorescent indicator that is based on homo FRET (fluorescence resonance energy transfer) using fluorescent molecules with a small stokes' shift, thereby developing a visualization system for intermolecular interaction. The present invention provides a fluorescent indicator formed by fusing fluorescent molecular components having substantially identical fluorescent properties to the N- and C-terminal sides of a target sequence, to which an analytical substance binds or reacts, so as to change the three-dimensional structure of the indicator.

# Fig. 1

Venus —— Asp — Glu — Val — Asp — Venus

Venus —— calmodulin —— M13 —— Venus

EP 1 571 448 A1

**Description**

TECHNICAL FIELD

The present invention relates to a fluorescent indicator used for analyzing intermolecular interaction using fluorescence resonance energy transfer (FRET), and a use thereof. More specifically, the present invention relates to a method for analyzing intermolecular interaction, which is characterized in that FRET generated as a result of the interaction between a fluorescent indicator formed by fusing two fluorescent molecules having substantially identical fluorescent properties to each other via a target sequence and an analytical substance is measured based on depolarization.

BACKGROUND ART

Fluorescence analysis method of a biological system is more advantageous than other biological methods in that this analysis method can noninvasively be carried out. With the development of physical fluorescence analyses, a biological reporter construct used as a monitor for a reaction performed in a cell has been developed, for example. In particular, if a fluorescent protein that does not need a cofactor for its specific fluorescence were developed, various analyses could be carried out by introducing such a protein into a cell via a gene construct and by allowing it to express therein. A green fluorescent protein (GFP) derived from *Aequorea victoria* has particularly been known as such a fluorescent protein. Miyawaki et al., Nature, 388, 882-887, 1997, describes a $Ca^{2+}$ sensing system that is based on GFP.
An example of a method for analyzing a biological system (in particular, analysis of an interaction between molecules, etc.) based on FRET is described in Miyawaki et al., Nature, 388, 882-887, 1997. However, all of the conventional FRET methods using fluorescent molecules have used two types of fluorescent molecules having different colors (spectra). Such methods have used a considerable part of a visible region for a single FRET observation. Thus, the conventional methods have been problematic in that a simultaneous observation of other fluorescent dyes is limited, for example.

DISCLOSURE OF THE INVENTION

It is an object of the present invention to provide a fluorescent indicator capable of simply and visibly detecting the interaction between a target protein and an analytical substance, and a method of using the above fluorescent indicator. More specifically, it is an object of the present invention to provide a fluorescent indicator that is based on homo FRET (fluorescence resonance energy transfer) using fluorescent molecules with a small stokes' shift, thereby developing a visualization system for intermolecular interaction.
As a result of intensive studies directed towards achieving the aforementioned objects, the present inventors have found that an analytical substance can be detected and measured by using a fluorescent indicator formed by fusing fluorescent molecular components having substantially identical fluorescent properties to the N- and C-terminal sides of a target sequence, to which the analytical substance binds or reacts, so as to change the three-dimensional structure of the indicator. The present invention has been completed based on these findings.
That is to say, the present invention provides a fluorescent indicator formed by binding fluorescent molecular components having substantially identical fluorescent properties to the N- and C-terminal sides of a target sequence, to which an analytical substance binds or reacts, so as to change the three-dimensional structure of the indicator.
The present invention preferably provides a fluorescent indicator, which comprises:

a target sequence, to which an analytical substance binds or reacts, so as to change the three-dimensional structure of the indicator;
a donor fluorescent molecular component that covalently fused to the target sequence; and
an acceptor fluorescent molecular component that covalently fused to the target sequence,

wherein the donor fluorescent molecule and the acceptor fluorescent molecule have substantially identical fluorescent properties, and wherein the three-dimensional structure of the target sequence is changed due to the analytical substance binding to the target sequence, and the relative positions or directions of the donor and the acceptor molecular component are then changed, and it is highly likely that the polarization properties of fluorescence observed when such a fluorescent molecule is excited by irradiation light having certain polarization properties differ from those of the irradiation light (depolarization).
Preferably, the fluorescence molecular component is a fluorescent protein or a mutant thereof.
Preferably, the fluorescence molecular component is a yellow fluorescent protein or a mutant thereof.
Preferably, the fluorescence molecular component is a fluorescent protein Venus.

Preferably, the fluorescent indicator further comprises a target peptide component and a linker component, wherein the target sequence of the analytical substance further comprises a peptide-binding domain for allowing the target peptide component to bind thereto,

wherein the linker component allows the target sequence of the analytical substance to covalently fuse to the target peptide component, and the target sequence and the target peptide component covalently fuse to either the acceptor fluorescent molecular component or the donor fluorescent molecular component, and

wherein the analytical substance binding to the target sequence induces a change in the relative positions or directions of the target peptide component and the peptide-binding domain, and the relative positions or orientation of the donor and the acceptor molecular component are then changed, and it is thereby highly likely that the polarization properties of fluorescence observed when such a fluorescent molecule is excited by irradiation light having certain polarization properties differ from those of the irradiation light (depolarization).

The target sequence is preferably calmodulin, cGMP-dependent protein kinase, a steroid hormone receptor, a ligand-binding domain of a steroid hormone receptor, protein kinase C, inositol-1,4,5-triphosphate receptor, or recobelin.

The target sequence of the analytic substance is preferably calmodulin.

The target peptide component is preferably skeletal muscle myosin light chain kinase (skMLCKp), smooth muscle myosin light chain kinase (smMLCK), calmodulin kinase II (CaMKII), caldesmon, calspermine, phosphofructokinase, calcineurin, phosphorylase kinase, $Ca^{2+}$-ATPase, 59 Kda phosphodiesterase (PDE), 60 Kda phosphodiesterase (PDE), nitric oxide synthase, type I adenylyl cyclase, *Bordetella pertussis* adenylyl cyclase, neuromodulin, spectrin, myristoylated alanine-rich C kinase substrate (MARCKS), MacMARCKS(F52), b-Adducin, heat shock protein HSP90a, human immunodeficiency virus envelope glycoprotein 160 (HIV-1 gp160), brush-boarder myosin heavy chain-I (BBMHBI), dilute myosin heavy chain (MHC), mastoparan, melittin, glucagon, secretin, vasoactive intestinal peptide (VIP), gastrin inhibitory peptide (GIP), or a calmodulin-binding domain of calmodulin-binding peptide-2 (Model peptide CBP2).

The linker component is preferably a peptide component.

The linker component preferably consists of 1 to 30 amino acid residues.

The target sequence, on which an analytical substance acts, so as to change the three-dimensional structure of the indicator, is preferably an amino acid sequence that is cleaved with enzymes.

The fluorescent indicator of the present invention is preferably a single polypeptide.

The fluorescent indicator of the present invention preferably further comprises a localized sequence.

The localized sequence is preferably a nucleus-localized sequence, an endoplasmic reticulum-localized sequence, a peroxisome-localized sequence, a mitochondrion-localized sequence, a Goldi apparatus-localized sequence, or a cell membrane-localized sequence.

In another aspect, the present invention provides a method for detecting or measuring an analytical substance in a sample, which comprises:

(1) a step of allowing a sample to interact with the fluorescent indicator of the present invention;
(2) a step of exciting a donor component; and
(3) a step of measuring the level of fluorescence resonance energy transfer in the indicator that reflects the concentration or activity of the analytical substance in the sample.

The level of fluorescence resonance energy transfer in the indicator is preferably measured by depolarization.

Such depolarization is preferably measured by obtaining fluorescence anisotropy.

The sample is preferably a living cell, and the above step comprises incorporation of the fluorescent indicator into the cell.

Such a step of incorporating the fluorescent indicator into a cell preferably comprises transfection of the cell with an expression vector containing an expression regulatory sequence that is functionally ligated to a nucleic acid sequence encoding the expression of the fluorescent indicator.

In another aspect, the present invention provides nucleic acid encoding the fluorescent indicator of the present invention, an expression vector containing the above nucleic acid, and a transformant having the above nucleic acid or expression vector.

BRIEF DESCRIPTION OF THE DRAWINGS

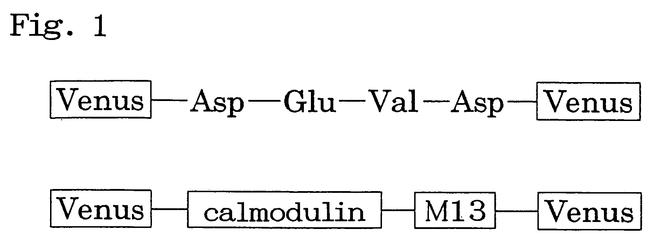

Figure 1 shows the structure of W-cameleon and that of W-SCAT, which are the fluorescent indicators of the present invention.

BEST MODE FOR CARRYING OUT THE INVENTION

The embodiments of the present invention will be described in detail below.

The fluorescent indicator of the present invention is formed by fusing fluorescent molecular components having substantially identical fluorescent properties to the N- and C-terminal sides of a target sequence, to which an analytical substance binds or reacts, so as to change the three-dimensional structure of the indicator. More specifically, the fluorescent indicator of the present invention comprises:

a target sequence, to which an analytical substance binds or reacts, so as to change the three-dimensional structure of the indicator;
a donor fluorescent molecular component that covalently fuses to the target sequence; and
an acceptor fluorescent molecular component that covalently fuses to the target sequence,

which is characterized in that the donor and the acceptor fluorescent molecule have substantially identical fluorescent properties, and characterized in that the three-dimensional structure of the target sequence is changed due to the analytical substance binding to the target sequence, and the relative positions or orientation of the donor and the acceptor molecular component are then changed, and also characterized in that it is highly likely that the polarization properties of fluorescence observed when such a fluorescent molecule is excited by irradiation light having certain polarization properties differ from those of the irradiation light (depolarization).

In a case where a fluorescent molecule is excited with light polarized in the vertical direction, after a chromophoric group of the fluorescent molecule had been excited, fluorescence emission has completed before the fluorescent molecule rotates. Thus, fluorescence polarized in the vertical direction is generally detected. However, if the same fluorescent molecule exists near the above fluorescent molecule, FRET occurs, and deporalization of the observed fluorescence thereby occurs. Such FRET (homotransfer) occurring between the same fluorescent molecules can be measured by depolarization method. In order to realize homotransfer FRET, it is necessary that the excitation spectrum of a fluorescent molecule highly overlap with the fluorescence spectrum thereof (a small Stoke's shift). GFP mutants such as CFP, YFP, or RFP are suitable.

In the present invention, a substrate site of protease, or a domain whose structure is altered with $Ca^{2+}$, is used as a target sequence, and the same fluorescent molecules are allowed to bind to the N- and C-terminal sides of such a target sequence, so as to produce a fluorescent indicator. Using such a fluorescent indicator, it becomes possible to monitor the activity of caspase 3 associated with apoptosis or a change in intracellular $Ca^{2+}$ concentration.

The term "fluorescent molecule" is used in the present invention to mean any given molecule capable of emitting fluorescence, when it is excited with appropriate electromagnetic radiations. For example, a fluorescent protein can be used as a fluorescent molecule.

In the present invention, the donor and the acceptor fluorescent molecular component have substantially identical fluorescent properties. The two molecular components are preferably identical fluorescent molecules. The fluorescent molecule used in the present invention is preferably selected in terms of FRET efficiency. Such FRET efficiency can be examined according to the techniques described in the present specification and the techniques widely known to persons skilled in the art.

The term "covalently fuses" is used to mean a covalent bond, or other covalent connections between two molecules. An example of such a covalent connection may be a divalent component for connecting two molecules.

The term "target sequence" is used to mean an amino acid sequence capable of binding to an analytical substance. When a preferred target sequence binds to an analytical substance, its three-dimensional structure is changed. The term "target peptide" is used to mean a peptide capable of binding to a target sequence. Such a target peptide is a partial sequence of a peptide binding to the target sequence.

The term "analytical substance" is used to mean a molecule or ion in a solution binding to a target sequence. Such an analytical substance changes the three-dimensional structure of a target sequence. An analytical substance may bind to a target sequence either reversibly or irreversibly.

The term "component" is used to mean a group of a molecule that fuses to another group of the indicator. That is, the "fluorescent molecular component" is a group of a fluorescent molecule fusing to a target sequence component or a linker component. The term "target sequence component" is used to mean a group of a target sequence fusing to a fluorescent molecular component. The term "target peptide component" is used to mean a group of a target peptide of a target sequence. The term "linker component" is used to mean a group of a molecular linker fusing to both donor and acceptor fluorescent molecular components.

The term "functionally ligated" is used to mean that a certain component is positioned such that a target component can function. A regulatory sequence is functionally ligated to a coding sequence, so that the expression of the coding sequence can be achieved under conditions that are suitable for the regulatory sequence. The term "regulatory sequence" is used to mean a polynucleotide necessary for the expression of a coding sequence and a non-coding sequence, to which the regulatory sequence is ligated. Examples of a regulatory sequence include a promoter, a ribosome-binding site, and a transcription termination sequence. Such a regulatory sequence may further comprise a leader sequence and the sequence of the other partner of a fusion protein.

The term "polynucleotide" is used to mean a nucleotide having a length of at least 10 nucleotides. Such a nucleotide may be ribonucleotide, deoxynucleotide, or a modified body thereof. Also, it may be either a single strand or a double strand.

The fluorescent indicator of the present invention, which uses fluorescence resonance energy transfer (FRET) to detect or measure an analytical substance, comprises two fluorescent molecular components having essentially same emission and excitation spectra, which provide a donor and an acceptor fluorescent molecular component. The donor and acceptor fluorescent molecular component used in the present invention have substantially identical fluorescent properties. They are preferably identical components. The two fluorescent components are selected, such that the excitation spectrum of the acceptor fluorescent molecular component highly overlaps with the emission spectrum of the donor fluorescent molecular component. The donor and acceptor fluorescent molecular components (these components have substantially identical fluorescent properties in the present invention) fuse to a target sequence component, the three-dimensional structure of which is changed by an analytical substance binding thereto. The relative positions and orientation of the donor and acceptor fluorescent molecules are changed due to the change in the above three-dimensional structure, and depolarization thereby occurs.

Such a fluorescent molecular component preferably covalently fuses to the amino terminus and carboxy terminal of the target sequence component. With such a configuration, the donor and acceptor fluorescent molecular components can move closely to each other, when an analytical substance binds thereto. Otherwise, the donor and acceptor components may also move such that they are separated from each other, when an analytical substance binds thereto. For example, such an acceptor component covalently fuses to a target peptide component binding to a target sequence component, and the target peptide component covalently fuses to the target sequence component via a linker component. Such a linker component is flexible, and the target peptide component can fuses to the target sequence component via the linker component. The donor component is excited with light having appropriate intensity in the excitation spectrum thereof. The donor component emits the absorbed energy in the form of fluorescence. When the acceptor fluorescent molecular component exists at the position where it is able to quench the donor component in an exited state, the fluorescence energy is transferred to the acceptor component, thereby emitting fluorescence.

In the present invention, FRET (which is referred to as homo FRET in the present specification) is promoted by using a single type of fluorescent molecules (that is, fluorescent molecules having substantially identical fluorescent properties). Homo FRET can generally be observed by measuring the depolarization of fluorescence. Factors for promoting the depolarization of fluorescence include reabsorption of fluorescence, FRET, and the rotatory Brownian motion of fluorescence molecules. However, even under intracytoplasmic circumstances, which are considered to have the highest degree of freedom, the rotational relaxation time of a fluorescent protein such as GFP has been measured to be between 30 and 40 nsec. This is much longer than the lifetime of fluorescence of GFP (3 or 4 nsec). Thus, it is extremely unlikely that a chromophoric group itself of a fluorescent molecule rotates at a high speed that is enough for depolarization. In addition, there is almost no influence due to reabsorption of fluorescence under conditions where GFP is sufficiently diluted, or under circumstances where a light path length is extremely short, such as in a cell. Accordingly, when depolarization is observed in fluorescence emitted from fluorescent molecules, it can be concluded that FRET occurs.

The intensity of polarization can be evaluated using fluorescence anisotropy. Such fluorescence anisotropy (Anisotropy: A) can be obtained with the following formula:

$$A = [I \text{ (parallel)} - I \text{ (vertical)}] / [I \text{ (parallel)} + 2 \times I \text{ (vertical)}]$$

wherein I (parallel) indicates a fluorescent component vector that is parallel to the polarization direction of incident light, and I (vertical) indicates a fluorescent component vector that is vertical to the polarization direction of incident light.

Fluorescence in a sample can be measured by using a fluorescence polarization degree measurement device. In general, an excitation radiation generated from an excitation source passes through an excitation optical system. The excitation radiation excites the sample through the excitation optical system. In response to this, fluorescence molecules in the sample emit a luminescence that is different from an excitation wavelength. Subsequently, a recovery optical system recovers the radioactive ray emitted from the sample. This device has a temperature controller for maintaining the sample at a constant temperature during scanning. As an example, a microtiter plate for remaining a plurality of samples moves by a multiaxial conversion stage. A multiaxial conversion stage, a temperature controller, an automatic focusing function, and an electronic device associated with imaging and data collection, are controlled by a digital computer that is appropriately programmed.

For example, the system for an efficient detection of depolarization due to homo FRET in vitro (e.g. in a cubette or plate) and in vivo (e.g. under a microscope or in a cell) can be set up.

Measurement of a fluorescence anisotropy can be carried out by using a commercially available device such as BEACON (TAKARA). A method for measuring a fluorescence anisotropy has already been known to persons skilled in the art. It is described in *Tanpakushitsu, nucleic acid, koso* (Proteins, nucleic acids, and enzymes), Vol. 42, No. 1, pp. 77-81, 1997, for example.

In the present invention, any given fluorescent molecule can be used. For example, a green fluorescent protein (GFP) derived from cnidarians and a mutant thereof can be used. Examples of such a mutant include a cyan fluorescent protein (CFP), a yellow fluorescent protein (YFP), a red fluorescent protein (RFP), and a blue fluorescent protein (BFP). These fluorescent proteins are obtained from Pacific Northwest jellyfish, *Aequorea victoria*, the sea pansy, *Renilla reniformis,* and *Phialidium gregarium*, etc. (Ward, W. W. et al., Photochem. Photobiol., 35: 803-808 (1982); and Levine, L. D. et al., Comp. Biochem. Physiol., 72B: 77-85 (1982)). Also, low molecular weight organic compounds such as fluorescein, rhodamine, Alexa, or Cy can also be used.

Fluorescent proteins having useful excitation and radiation spectra derived from various types of jellyfishes are produced by modifying the amino acid sequence of natural GFP derived from *Aequorea victoria* (Prasher, D. C. et al., Gene, 111: 229-233 (1992); Heim, R. et al., Proc. Natl. Acad. Sci., USA, 91:12501-04 (1994); US Patent Application No. 08/337,915; International Application PCT/US95/14692; and US Patent Application No. 08/706,408). GFP cDNA may be ligated to cDNA encoding other proteins described above. There may be cases where the obtained fusion body is also a fluorescent body, which retains the biochemical properties of the other protein (Cubitt, A. B. et al., Trends Biochem. Sci. 20: 448-455 (1995)). Moreover, a GFP mutant whose excitation or emission wavelength is shifted has been produced by mutagenesis (Heim, R. & Tsien, R. Y. Current Biol. 6:178-182 (1996)). A protein containing 150 contiguous amino acids having homology of 85% or more with the amino acid sequence of a wild-type fluorescent protein derived from a jellyfish is considered to be a fluorescent protein derived from the jellyfish.

The expression "a green fluorescent protein (GFP), a yellow fluorescent protein (YFP), or a mutant thereof" does not only mean known green fluorescent proteins and known yellow fluorescent proteins, but also means that all the mutants thereof are included. For example, a green fluorescent protein gene has been isolated and sequenced (Prasher, D.C. et al., "Primary structure of the *Aequorea victoria* green fluorescent protein," Gene 111: 229-233, (1992)). The amino acid sequences of many other fluorescent proteins and mutants thereof have also been reported. Such proteins and mutants are described in Roger Y. Tsin, Annu.Rev.Biochem.1998. 67: 509-44, and in the cited publications thereof, for example.

As such a green fluorescent protein (GFP), a yellow fluorescent protein (YFP), or a mutant thereof, those derived from *Aequorea victoria* can be used, for example.

Several examples of such GFP, YFP, and a mutant thereof are given below. It is to be noted that the expression "F99S" indicates that the amino acid residue F at position 99 is substituted with S. Substitutions of other amino acids are shown in the same above manner.

Wild type GFP;
GFP having amino acid mutations, F99S, M153T, and V163A;
GFP having amino acid mutation S65T;
GFP having amino acid mutations, F64L and S65T;
GFP having amino acid mutations, S65T, S72A, N149K, M153T, and I167T;
GFP having amino acid mutations, S202F and T203I;
GFP having amino acid mutations, T203I, S72A, and Y145F;
GFP having amino acid mutations, S65G, S72A, and T203F (YFP);
GFP having amino acid mutations, S65G, S72A, and T203H (YFP);
GFP having amino acid mutations, S65G, V68L, Q69K, S72A, and T203Y (EYFP-V68L, Q69K);
GFP having amino acid mutations, S65G, S72A, and T203Y (EYFP); and
GFP having amino acid mutations, S65G, S72A, K79R, and T203Y (YFP).

In the present invention, GFP mutants, such as CFP, YFP, REP, or a mutant thereof, are preferably used. For example, Venus that is a YFP mutant can be used. Please refer to Nagai, T. et al., Nature Biotecnology 20, 87-90, 2002, for Venus. Venus is a fluorescent protein obtained by substituting phenylalanine at position 46 of YFP with leucine. This protein achieves brightness that is 30 to 100 times greater than the conventional GFP in *Escherichia coli.* It achieves brightness that is 3 to 100 times greater than the conventional GFP in mammalian cells. Thus, Venus provides fluorescence that can sufficiently be detected with a common device.

Other fluorescent molecules that can be used in the present invention include: a yellow fluorescent protein derived from *Vibrio fischeri* Y-1 strain; Peridinin-chlorophyll (a protein derived from dinoflagellate Symbiodinium sp.); a phycobili protein derived from marine cyanobacteria such as Synechoccus (e.g. phycoerythrin and phycocyanin); and oat phytochrome derived from oats, which is reconstituted with phycoerythrobilin. These fluorescent proteins are described in Baldwin, T. O. et al., Biochemistry 29: 5509-5515 (1990), Morris, B. J. et al., Plant Molecular Biology, 24: 673-677 (1994), Wilbanks, S. M. et al., J. Biol. Chem. 268: 1226-1235 (1993), and Li et al., Biochemistry 34: 7923-7930 (1995), etc.

The FRET efficiency obtained between the donor and acceptor fluorescent molecular components can be regulated by controlling the ability of the two fluorescent molecules to interact with each other. The properties of the target sequence component, target peptide component, and linker component also have effects on FRET and the response of the indicator to the analytical substance. Generally, it is desired that a significant change occur in the three-dimensional structure of the target sequence component.

The target sequence component is a protein the three-dimensional structure of which is changed due to the binding of the analytical substance, or a portion thereof. Examples of such a protein include calmodulin (CaM), cGMP-dependent protein kinase, a steroid hormone receptor (or a ligand-binding domain thereof), protein kinase C, an inositol-1,4,5-tirphosphate receptor, and recobelin (refer to Katzenellenbogen, J. A. & Katzenellenbogen, B. S. Chemistry & Biology 3: 529-536 (1996), and Ames, J. B. et al., Curr. Opin. Struct. Biol. 6: 432-438 (1996), for example). The target sequence component preferably binds to the target peptide, as well as the analytical substance.

The target peptide component includes any given amino acid sequences shown in Table 1 and portions thereof. However, the target peptide should be able to bind to the target sequence component. The target peptide may also be a partial sequence of a calmodulin-binding domain. The target peptide components shown in Table 1 are recognized by the target sequence component CaM (refer to Crivici, A. & Ikura, M. Annu. Rev. Biophys. Biomol. Struct. 24: 84-116 (1995), for example). The response of the fluorescent indicator to the analytical substance may be reinforced by modification of the target peptide component. Other target peptide components binding to other target sequences are already known to persons skilled in the art.

Table1

| Target | Sequence |
|---|---|
| SkMLCK (M13) | KRRWKKNFIAVSAANRFKKISSSGAL (SEQ ID NO: 1) |
| smMLCK (smMLCKp) | ARRKWQKTGHAVRAIGRLSS (SEQ ID NO: 2) |
| CaMKII | ARRKLKGAILTTMLATRNFS (SEQ ID NO: 3) |
| Caldesmon | GVRNIKSMWEKGNVFSS (SEQ ID NO: 4) |
| Calspermin | ARRKLKAAVKAVVASSRLGS (SEQ ID NO: 5) |
| PFK (M11) | FMNNWEVYKLLAHIRPPAPKSGSYTV (SEQ ID NO: 6) |
| Calcineurin | ARKEVIRNKIRAIGKMARVFSVLR (SEQ ID NO: 7) |
| PhK (PhK5) | LRRLIDAYAFRIYGHWVKKGQQQNRG (SEQ ID NO: 8) |
| (PhK13) | RGKFKVICLTVLASVRIYYQYRRVKPG (SEQ ID NO: 9) |
| $Ca^{2+}$-ATPase (C28W) | LRRGQILWFRGLNRIQTQIKVVNAFSSS (SEQ ID NO: 10) |
| 59-kDa PDE | RRKHLQRPIFRLRCLVKQLEK (SEQ ID NO: 11) |
| 60-kDa PDE | TEKMWQRLKGILRCLVKQLEK (SEQ ID NO: 12) |
| NOS (NO-30) | KRRAIGFKKLAEAVKFSAKLMGQ (SEQ ID NO: 13) |
| Type I AC (AC-28) | IKPAKRMKFKTVCYLLVQLMHCRKMFKA (SEQ ID NO: 14) |
| *Bordetella pertussis* AC | IDLLWKIARAGARSAVGTEA (SEQ ID NO: 15) |

| Neuromodulin | KAHKAATKIQASFRGHITRKKLKGEKK (SEQ ID NO: 16) |
| Spectrin | KTASPWKSARLMVHTVATFNSIKE (SEQ ID NO: 17) |
| MARCKS | KKKKKRFSFKKSFKLSGFSFKKSKK (SEQ ID NO: 18) |
| F52 or MacMARKS | KKKKKFSFKKPFKLSGLSFKRNRK (SEQ ID NO: 19) |
| β -Adducin | KQQKEKTRWLNTPNTYLRVNVADEVQRNMGS (SEQ ID NO: 20) |
| HSP90a | KDQVANSAFQERLRKHGLEVI (SEQ ID NO: 21) |
| HIV-1 gp160 | YHRLRDLLLIVKRIVELLGRR (SEQ ID NO: 22) |
| BBMHBI | QQLATLIQKTYRGWRCRTHYQLM (SEQ ID NO: 23) |
| Dilute MHC | RAACIRIQKTIRGWLLRKRYLCMQ (SEQ ID NO: 24) |
| Mastoparan | INLKAALAKKIL (SEQ ID NO: 25) |
| Melittin | GIGAVLKVLTTGLPALISWIKRKRQQ (SEQ ID NO: 26) |
| Glucagon | HSQGTFTTSDYSKYLDSRRAQDFVQWLMNT (SEQ ID NO: 27) |
| Secretin | HSDGTFTSELSRLRDSARLQRLLQGLV (SEQ ID NO: 28) |
| VIP | HSDAVFTDNYTRLRKQMAVKKYLNSILN (SEQ ID NO: 29) |
| GIP | YADGTFISDYSAIMNKIRQQDFVNWLLAQQQKS (SEQ ID NO: 30) |
| Model peptide CBP2 | KLWKKLLKLLKKLLKLG (SEQ ID NO: 31) |

Explanation of abbreviated expressions

AC: adenylyl cyclase;

BBMHCI: brush-border myosin heavy chain-I;

CaMKII: calmodulin kinase II;

CBP2: calmodulin-binding peptide-2;

GIP: gastrin inhibitory peptide;

HIV-1 gp160: human immunodeficiency virus envelope glycoprotein 160;

HSP: heat shock protein;

MARCKS: myristoylated alanine-rich C kinase substrate;

MHC: myosin heavy chain;

NOS: nitric oxide synthase;

PDE: phosphodiesterase;

PFK: phosphofructokinase;

PhK: phosphorylase kinase;

sk-, smMLCK: skeletal muscle and smooth muscle myosin light chain kinase; and

VIP: vasoactive intestinal peptide.

The length of a linker component is selected, such that it optimizes FRET and the speed and specificity regarding a change in the three-dimensional structure due to the binding of the analytical substance. Such a linker component preferably has a length and flexibility that are necessary for free interaction between the target sequence component and the target peptide component, which is capable of responding the concentration or activity of the analytical substance. In order to optimize FRET effects, the mean distance between the donor and acceptor fluorescent molecular components is preferably between approximately 1 nm and approximately 10 nm, more preferably between approximately 1 nm and approximately 6 nm, and particularly preferably between 1 nm and approximately 4 nm. If such a linker molecule is too short or too solid, the donor and acceptor molecular components cannot easily change the positions. In contrast, if such a linker molecule is too long, the target peptide component cannot efficiently bind to the target sequence component. Such a linker component is preferably a peptide component. A preferred linker component is a peptide consisting of 1 to 30 amino acid residues, and preferably 1 to 15 amino acid residues. The -Gly-Gly- linker is an example of such a linker.

A linker component may comprise a flexible spacer amino acid sequence. Such a linker component is described in Huston, J. S. et al., PNAS 85: 5879-5883 (1988), Whitlow, M. et al., Protein Engineering 6: 989-995 (1993), and Newton, D. L. et al., Biochemistry. 35: 545-553 (1996), for example.

Any target sequence may be used, as long as an analytical substance can bind to or act on the target sequence, so as to change the three-dimensional structure of an indicator. For example, a sequence that is recognized and cleaved with enzymes may also be used. For example, a substrate site of protease can be used as such a target sequence. When caspase 3 is used as protease, DEVD can be used as the amino acid sequence of a target sequence.

The fluorescent indicator may comprise a localized sequence. Through such a localized sequence, the indicator is fused with a preferred intracellular organelle target signal or with a localized host protein, and it is thereby transferred to a specific site in a cell. A polynucleotide encoding such a localized sequence or signal sequence can be ligated to or fused to the 5'-terminus of a polynucleotide encoding the fluorescent indicator, so that a signal peptide can be positioned at the amino terminus of the obtained fusion polynucleotide or polypeptide.

In the case of a eukaryotic cell, it is considered that a signal peptide has a function of transporting a fusion polypeptide via the endoplasmic reticulum. The secretory protein is then transferred to the Goldi apparatus, and then to the secretory vesicle and the extracellular space, and preferably to the external environment. The signal peptide usable in the present invention may be a prepropeptide containing a proteolytic enzyme recognition site.

Such a localized sequence may be a nucleus-localized sequence, an endoplasmic reticulum-localized sequence, a peroxisome-localized sequence, a mitochondrion-localized sequence, or a localized protein. For example, the target sequence described in "Protein Targeting," Chapter 35, Stryer, L., Biochemistry (4th ed.). W. H. Freeman, 1995, may also be used as a localized sequence. Such a localized sequence may also be a localized protein. Specific examples of such a localized sequence include: a sequence targeting for the nucleus ((KKKRK) (SEQ ID NO: 32); a sequence targeting for the mitochondria (wherein the amino terminus is MLRTSSLFTRRVQPSLFRNILRLQST-) (SEQ ID NO: 33); a sequence targeting for the endoplasmic reticulum (KDEL (SEQ ID NO: 34) at the C-terminus) (wherein the signal sequence exists at the N-terminus); a sequence targeting for the peroxisome (SKF (SEQ ID NO: 35) at the C-terminus); a sequence targeting for prenylation or insertion into a cell membrane ([CaaX] CAAX (SEQ ID NO: 36), CC (SEQ ID NO: 37), CXC (SEQ ID NO: 38), or CCXX (SEQ ID NO: 39) at the C-terminus); a sequence targeting for the cytoplasmic side of a cell membrane (fusion to SNAP-25); and a sequence targeting for the Goldi apparatus (fusion to furin).

A fluorescent indicator can be produced in the form of a fusion protein by recombinant DNA techniques. The production of a fluorescent protein by recombination is carried out via the expression of nucleic acid encoding the protein. Such nucleic acid encoding the fluorescent protein can be obtained by methods that have already been known to persons skilled in the art. For example, nucleic acid encoding a protein can be isolated from cDNA derived from *Aequorea victoria* by PCR using primers based on the DNA sequence of an *Aequorea victoria* green fluorescent protein. Various mutants of such a fluorescent protein can be produced by performing site-directed mutagenesis or random mutagenesis on nucleic acid encoding the fluorescent protein. Random mutagenesis can be carried out by performing PCR, using 0.1 mM MnCl or while destroying the balance of a nucleotide concentration.

The construction of an expression vector and the expression of a gene in cells transfected therewith can be carried out according to molecular cloning methods known to persons skilled in the art. The details of such molecular cloning methods are described in Sambrook et al., Molecular Cloning-A Laboratory Manual, Cold Spring Harbor Laboratory, Cold Spring Harbor, NY, (1989), and Current Protocols in Molecular Biology, F. M. Ausubel et al., eds., (Current Protocols, a joint venture between Greene Publishing Associates, Inc. and John Wiley & Sons, Inc., most recent Supplement).

Nucleic acid used for transfection of cells with a sequence encoding the expression of a polypeptide is generally an expression vector containing an expression regulatory sequence that is functionally ligated to a nucleotide sequence encoding the expression of a polypeptide. The expression "a nucleotide sequence encoding the expression of a polypeptide" is used herein to mean a sequence that generates a polypeptide as a result of the transcription and translation of mRNA. For example, a sequence containing intron is included in such a nucleotide sequence. The term "expression regulatory sequence" is used herein to mean a nucleic acid sequence that regulates the expression of nucleic acid, to which the nucleic acid sequence is functionally ligated. When such an expression regulatory sequence regulates and controls the transcription and translation of a nucleic acid sequence, it is functionally ligated to the nucleic acid sequence. Such an expression regulatory sequence may comprise a suitable promoter, enhancer, transcription terminator, initiation codon located before a protein-coding gene (that is, ATG), a splicing signal of intron, a stop codon, or the like.

An expression vector containing the coding sequence of a fluorescent indicator and an appropriate transcription and translation regulatory signal can be constructed by methods widely known to persons skilled in the art. Examples of such methods include *in vitro* recombination DNA technique, synthesis technique, *in vivo* recombination technique, and genetic engineering technique (refer to techniques described in Maniatis et al., Molecular Cloning A Laboratory Manual, Cold Spring Harbor Laboratory, N.Y, 1989, for example). Transformation of host cells with recombinant DNA can be carried out by common techniques that have widely been known to persons skilled in the art. When host cells are prokaryotic cells such as *Escherichia coli,* competent cells capable of taking in DNA can be produced from cells, which are recovered after the logarithmic growth phase and then treated by the $CaCl_2$ method widely known to persons skilled in the art. Otherwise, $MgCl_2$ or RbCl can also be used. Transformation can be carried out after the production of protoplasts of host cells, or by electroporation.

When host cells are eukaryotic cells, the calcium phosphate coprecipitation method, microinjection, electroporation, or the DNA transfection method such as insertion of a plasmid encapsulated into a liposome or a viral vector, can be applied. Eukaryotic cells can be transfected with a DNA sequence encoding the fusion polypeptide of the present invention and a foreign DNA molecule encoding an appropriate phenotype such as a herpes simplex thymidine kinase gene. Also, eukaryotic cells are transiently infected or transformed with a eukaryotic viral vector such as simian virus 40 (SV40) or bovine papilloma virus, so as to allow a protein to express therein (refer to Eukaryotic Viral Vectors, Cold Spring Harbor Laboratory, Gluzman ed., 1982). Preferably, eukaryotic cells are used as host cells.

As a method for isolating or purifying the polypeptide of the present invention that has been allowed to express in microorganisms or eukaryotic cells, any given common method can be used. For example, preparative chromatographic separation or immunological separation (including the use of a monoclonal or polyclonal antibody or an antigen) is applied.

In order to allow a sequence encoding the fluorescent indicator to express, various types of host/expression vector systems can be used. Examples of such a host/expression vector system include: bacteria transformed with a recombinant bacteriophage DNA, plasmid DNA, or cosmid DNA expression vector, which contains the sequence encoding the fluorescent indicator; yeast transformed with a recombinant yeast expression vector containing the sequence encoding the fluorescent indicator; plant cells infected with a recombinant viral expression vector containing the sequence encoding the fluorescent indicator (e.g. cauliflower mosaic virus (CaMV), tobacco mosaic virus (TMV)), or plant cells transformed with a recombinant plasmid expression vector (e.g. Ti plasmid) containing the sequence encoding the fluorescent indicator; an insect cell system infected with a recombinant viral expression vector (e.g. Baculovirus) containing the sequence encoding the fluorescent indicator; and an animal cell system infected with a recombinant viral expression vector (e.g. retrovirus, adenovirus, vaccinia virus) containing the sequence encoding the fluorescent indicator, but examples are not limited thereto.

Depending on the host/vector system used, appropriate transcription and translation elements (e.g. a constitutive or inducible promoter, a transcription-enhancer element, a transcription terminator, etc.) can be used in an expression vector (refer to Bitter et al., Methods in Enzymology 153: 516-544, 1987, for example). When an expression vector is cloned into bacteria for example, an inducible promoter, such as pL of bacteriophage λ, plac, ptrp, or ptac (a ptrp-lac hybrid promoter), can be used. When it is cloned into a mammalian cell system, a promoter derived from the genome of a mammalian cell (e.g. a metallothionein promoter) or a promoter derived from a mammalian virus (e.g. a retrovirus long terminal repeat, an adenovirus late promoter, a vaccinia virus 7.5 K promoter, etc.) can be used. It is also possible to transcribe an insertion sequence encoding the fluorescent indicator, using recombinant DNA or a promoter produced by synthesis techniques.

In the case of using bacteria, a large number of expression vectors can advantageously be selected depending on the intended use of the fluorescent indicator to be expressed therein. For example, when a large amount of fluorescent indicator is produced, it is preferable to use a vector that orders a large amount of expression of a fusion protein product that is easily purified. Such a vector is preferably processed such that it contains a cleavage site for supporting the recovery of the fluorescent indicator.

In the case of using yeast, a large number of vectors containing a constitutive or inducible promoter can be used. Refer to Current Protocols in Molecular Biology, Vol. 2, Ed. Ausubel et al., Greene Publish. Assoc. & Wiley Interscience, Ch. 13, 1988; Grant et al., Expression and Secretion Vectors for Yeast, in Methods in Enzymology, Eds. Wu & Grossman, 31987, Acad. Press, N.Y, Vol. 153, pp.516-544, 1987; Glover, DNA Cloning, Vol. II, IRL Press, Wash., D.C., Ch. 3, 1986; Bitter, Heterologous Gene Expression in Yeast, Methods in Enzymology, Eds. Berger & Kimmel, Acad. Press, N.Y., Vol. 152, pp. 673-684, 1987; and The Molecular Biology of the Yeast Saccharomyces, Eds. Strathern et al., Cold Spring Harbor Press, Vols. I and II, 1982, for example. A constitutive yeast promoter such as ADH or LEU2 or an inducible promoter such as GAL can be used (Cloning in Yeast, Ch. 3, R. Rothstein In: DNA Cloning Vol.11, A Practical Approach, Ed. DM Glover, IRL Press, Wash., D.C., 1986). Also, a vector that promotes incorporation of foreign DNA into the chromosome of yeast can be used.

In the case of using a plant expression vector, the expression of a sequence encoding the fluorescent indicator can be promoted with a promoter. For example, viral promoters such as CaMV 35S RNA and 19S RNA promoters (Brisson et al., Nature 310: 511-514, 1984) or the coat protein promoters of TMV (Takamatsu et al., EMBO J. 6:307-311, 1987) can be used. In addition, a plant promoter such as RUBISCO small subunit (Coruzzi et al., 1984, EMBO J. 3: 1671-1680; Broglie et al., Science 224: 838-843, 1984) or a heat shock promoter (e.g. soybean hsp 17.5-E or hsp 17.3-B (Gurley et al., Mol. Cell. Biol. 6: 559-565, 1986)) can also be used. These constructs can be introduced into plants using Ti plasmid, Ri plasmid, plant viral vector, direct DNA transformation, microinjection, electroporation, or the like. These techniques are described in Weissbach & Weissbach, Methods for Plant Molecular Biology, Academic Press, NY, Section VIII, pp. 421-463, 1988; and Grierson & Corey, Plant Molecular Biology, 2nd Ed., Blackie, London, Ch. 7-9, 1988, for example.

It is also possible to allow the fluorescent indicator to express in an insect system. For example, a foreign gene can be expressed by using *Autographa californica* nuclear polyhedrosis virus (AcNPV) as a vector. This virus grows in *Spodoptera frugiperda* cells. A sequence encoding the fluorescent indicator is cloned into the nonessential region of the above virus (e.g. a polyhedrosis gene), and it is placed under the control of the AcNPV promoter. When a sequence encoding the fluorescent indicator is correctly inserted, a polyhedrosis gene becomes inactivated, and a non-occlusive recombinant virus is generated. Thereafter, *Spodoptera frugiperda* cells are infected with such a recombinant virus, and the inserted gene can be allowed to express in the cells (refer to Smith et al., J. Viol. 46:584, 1983; and US Patent No. 4,215,051, for example).

Using a eukaryotic cell system, and preferably a mammalian cell expression system, it becomes possible to carry out a suitable post-translation modification on the expressed mammalian protein. Eukaryotic cells having a cell mechanism for the suitable processing, glycosylation, and phosphorylation of a primary transcript, and for the secretion of a gene product, can preferably be used as host cells for expression of the fluorescent indicator. Examples of such a host cell line include CHO, VERO, BHK, HeLa, COS, MDCK, Jurkat, HEK-293, and WI38, but examples are not limited thereto.

A mammalian cell line that orders expression by using a recombinant virus or viral element can be constructed. When an adenovirus expression vector is used for example, a sequence encoding the fluorescent indicator can be ligated to an adenovirus transcription and translation regulatory complex (e.g. a late promoter and 3 leader sequences, etc.) This chimeric gene can be inserted into adenovirus genome by *in vitro* or *in vivo* recombination. By inserting the gene into the nonessential region (e.g. E1 or E3 region) of the virus genome, a recombinant virus capable of surviving in the infected host and allowing the fluorescent indicator to express therein can be obtained (refer to Logan & Shenk, Proc. Natl. Acad. Sci. USA, 81: 3655-3659, 1984, for example). Otherwise, a vaccinia virus 7.5K promoter can be used (refer to Mackett et al., Proc. Natl. Acad. Sci. USA , 79: 7415-7419, 1982; Mackett et al., J. Virol. 49: 857-864, 1984; Panicali et al., Proc. Natl. Acad. Sci. USA 79: 4927-4931, 1982, for example). It is also possible to use a vector based on a bovine papilloma virus having replication ability as an extrachromosomal element (Sarver et al., Mol. Cell. Biol. 1: 486, 1981). Immediately after this DNA has been introduced into mouse cells, a plasmid replicates approximately 100 to 200 copies per cell. In order to transcribe the inserted cDNA, it is not necessary for the plasmid to be incorporated into the chromosome of the host. Thus, a high level of expression can be realized. By incorporating a selective marker such as a neo gene into a plasmid, these vectors can be used for stable expression. Alternatively, retrovirus genome is modified, and the modified genome can be used as a vector capable of inducing or ordering the expression of the fluorescent indicator gene in host cells (Cone & Mulligan, Proc. Natl. Acad. Sci. USA, 81: 6349-6353, 1984). A high level of expression can be achieved by using an inducible promoter such as a metalothionine IIA promoter or a heat shock promoter.

In order to produce a recombinant protein at a high yield for a long period of time, stable expression is preferable. Host cells can be transformed with the cDNA of a fluorescent indicator, which is regulated with suitable expression regulatory elements (e.g. a promoter, an enhancer sequence, a transcription terminator, a polyadenylation site, etc.) and a selective marker, instead of using an expression vector containing a replication origin of virus. A selective marker in a recombinant plasmid imparts a resistance to selection to the plasmid, so that a cell stably incorporates the plasmid into the chromosome thereof, and the cell then grows and forms a colony. The colony is then subjected to cloning, so as to establish a cell line. For example, after introduction of foreign DNA, the recombinant cells are allowed to proliferate in a rich medium for 1 or 2 days, and then the medium is exchanged with a selective medium. A large number of selective systems can be used. For example, a herpes simplex thymidine kinase gene (Wigler et al., Cell, 11: 223, 1977), a hypoxanthine guanine phosphoribosyltransferase gene (Szybalska & Szybalski, Proc. Natl. Acad. Sci. USA, 48:2026, 1962), and an adenine phosphoribosyltransferase gene (Lowy et al., Cell, 22: 817, 1980) are used in tk-, hgprt-, and aprt-cells, respectively. Moreover, antimetabolite resistance can be used as a base for the selection of a dhfr gene imparting a resistance to methotrexate (Wigler et al., Proc. Natl. Acad. Sci. USA, 77: 3567, 1980; O'Hare et al., Proc. Natl. Acad. Sci. USA, 8: 1527, 1981), a gpt gene imparting a resistance to mycophenolic acid (Mulligan & Berg, Proc. Natl. Acad. Sci. USA, 78: 2072, 1981), a neo gene imparting a resistance to aminoglucoside G-418 (Colberre-Garapin et al., J. Mol. Biol., 150:1, 1981), and a hygro gene imparting a resistance to hydromycin (Santerre et al., Gene, 30: 147, 1984).

In recent years, other selective genes have further been reported. Examples of such selective genes include: trpB that enables cells to use indole instead of tryptophan; hisD that enables cells to use histinol instead of histidine (Hartman & Mulligan, Proc. Natl. Acad. Sci. USA, 85:8047, 1988); and ODC (ornithine decarboxylase) that imparts a resistance to 2-(difluoromethyl)-DL-ornithine that is an ornithine decarboxylase inhibitor (McConlogue L., In: Current Communications in Molecular Biology, Cold Spring Harbor Laboratory, ed., 1987).

A DNA sequence encoding the polypeptide of the fluorescent indicator of the present invention can be allowed to express *in vitro* by introducing the DNA into suitable host cells. That is to say, the recombinant fluorescent protein of the present invention can be produced by allowing nucleic acid to express in prokaryotic cells such as *Escherichia coli* or in eukaryotic cells such as yeast or mammalian cells.

A construct may comprise a tag used for easy isolation of the fluorescent indicator. For example, a polyhistidine tag consisting of 6 histidine residues may be added to the amino terminus of the fluorescent protein. With such a polyhistidine tag, it becomes possible to easily isolate the protein in a single operation by nickel chelate chromatography.

The fluorescent indicator of the present invention is preferably a fusion protein produced by the recombinant DNA technique. Herein, a single polypeptide comprises a donor component, a peptide linker component, and an acceptor component. The donor component may be positioned on the amino terminal side with respect to the acceptor component in the polypeptide. Such a fusion protein generally has the following structure: (amino terminus) a donor fluorescent molecular component - a peptide linker component - an acceptor fluorescent molecular component (carboxy terminus). Alternatively, the donor component may also be positioned on the carboxy terminal side with respect to the acceptor component in the fusion protein. Such a fusion protein generally has the following structure: (amino terminus) an acceptor fluorescent molecular component - a peptide linker component - a donor fluorescent molecular component (carboxy terminus). Moreover, a fusion protein containing an amino acid sequence added to the amino terminus and/ or carboxy terminus (e.g. a polyhistidine tag, etc.) may also be included in the present invention.

The fluorescent indicator encoded by recombinant nucleic acid comprises a sequence encoding the expression of a donor fluorescent molecular component, an acceptor fluorescent molecular component, and a peptide linker component. Each constitutive element is selected, such that when a donor component is excited as a result of the expression of a fusion protein, donor and acceptor components exhibit FRET. Recombinant nucleic acid can be incorporated into an expression vector containing an expression regulatory sequence that is functionally ligated to the recombinant nucleic acid. An expression vector comprises a suitable promoter, replication sequence, marker, or the like, so that it can function in prokaryotic cells or eukaryotic cells.

Host cells can be transfected with such an expression vector, so as to allow recombinant nucleic acid to express therein. Host cells can be selected for a high level of expression of nucleic acid, from which a fluorescent indicator fusion protein is then purified. *Escherichia coli* (*E. coli*) is useful for such purpose. In addition, host cells may also be either other prokaryotic cells or eukaryotic cells. A linker peptide can be selected, such that it comprises an amino acid sequence recognized by protease. Cells may be either cultured cells or *in vivo* cells.

The present invention will be specifically described in the following examples. However, the examples are not intended to limit the scope of the present invention.

EXAMPLES

(1) Construction of W-cameleon gene

Using pRSET_B/Venus (Nagai, T et al., Nature Biotecnology 20, 87-90 (2002)) as a template, PCR was carried out with the following primers:

5'-attggatcccatggtgagcaagggcgagg-3 (SEQ ID NO: 40); and
5'-catgcatgcgggcggcggtcacgaactc-3' (SEQ ID NO: 41). The PCR product was then cleaved with restriction enzymes *Bam*HI and *Sph*I. pRSET_B/YC2.12 (Nagai, T et al., Nature Biotecnology 20, 87-90 (2002)) was cleaved with *Bam*HI and *Sph*I, so as to remove the ECFP gene, and the aforementioned PCR product treated with restriction enzymes was then ligated thereto, so as to obtain pRSET_B/W-cameleon that allows a W-cameleon protein to express in *Escherichia coli*. The structure of W-cameleon is shown in Figure 1 (lower case).

(2) Construction of W-SCAT gene

Using pRSET_B/Venus (Nagai, T et al., Nature Biotecnology 20, 87-90 (2002)) as a template, PCR was carried out with the following primers:

5'-gctggtaccatggtgagcaagggcgagg-3' (SEQ ID NO: 42);
5'-gcagaattctcacttgtacagctcgtccatgcc-3' (SEQ ID NO: 43). The PCR product was cleaved with restriction enzymes *Kpn*I and *Eco*RI. Thereafter, the resultant PCR product was ligated to the same restriction site of pRSETB, so as to obtain pRSETB/Venus-KE. Using pRSET_B/Venus (Nagai, T et al., Nature Biotecnology 20, 87-90 (2002)) as a template, PCR was carried out with the following primers:
5'-attggatcccatggtgagcaagggcgagg-3' (SEQ ID NO: 44); and
5'-gctggtaccatcgacctcatcagtgatcccggcggcggtcacgaa-3' (SEQ ID NO: 45). The PCR product was then cleaved with restriction enzymes *Bam*HI and *Kpn*I. This PCR product treated with restriction enzymes was ligated to the *Bam*HI-*Kpn*I site of pRSETB/Venus-KE, so as to obtain pRSET_B/W-SCAT that allows a W-SCAT protein to express in *Escherichia coli.* The structure of W-SCAT is shown in Figure 1 (upper case).

(3) Expression and purification of protein

*Escherichia coli* was transformed with pRSET_B/W-cameleon or pRSET_B/W-SCAT, and it was then cultured on an LB plate containing 50 µg/ml ampicillin at 37°C for 15 hours. A single colony was picked up and inoculated into a test tube that contained 20 ml of LB medium containing 50 µg/ml ampicillin, followed by culture at 200 rpm at room temperature for 4 days. After completion of the culture, *Escherichia coli* was recovered by centrifugation, and it was then dissolved in 10 ml of PBS(-). A cell body was disintegrated with a French press, and insoluble fractions were then removed by centrifugation. Thereafter, 800 µl of Ni-NTA agarose (QIAGEN) was added to the supernatant, and the mixture was blended at room temperature for 1 hour by turning it upside down. Thereafter, a protein was purified in accordance with protocols attached with the kit.

(4) Measurement of fluorescent anisotropy

The W-cameleon solution as obtained above was dissolved in 1 ml of measurement solution (50mM HEPES (pH7.5), 100 µM EGTA or 50mM HEPES (pH7.5), 100 µM EGTA, 1mM CaCl_2). The dilution ratio was set at 1/10,000. 2 µl of solution obtained by 10 times diluting the W-SCAT solution as obtained above and 1 unit of active caspase 3 (CPP32, MBL) were added to a buffer solution used for reaction (20mM HEPES (pH7.5), 100mM NaCl, 1mM EDTA, 10mM DTT, 10% sucrose), so as to obtain 20 µl of a mixed solution, followed by reaction at 37°C for 2 hours. A buffer solution to which no active caspase 3 had been added was used as a control. After completion of the reaction, 1 ml of PBS(-) was added to the reaction solution. The fluorescent anisotropy of each of the aforementioned proteins was measured using Beacon (TAKARA). The measurement results are shown in Table 2 indicated below. From the results shown in the table, it is found that an analytical substance contained in a sample can be detected or measured by using the fluorescent indicators of the present invention, W-cameleon and W-SCAT.

Table 2

| Measurement results of fluorescent anisotropy | | | |
|---|---|---|---|
| | Fluorescent component parallel to polarization direction of incident light | Fluorescent component vertical to polarization direction of incident light | Fluorescent anisotropy (mA) |
| W-SCAT3 | | | |
| without casp3 | 2555.7 | 1362.7 | 225.8 |
| With casp3 | 2712.5 | 1265.2 | 276.0 |
| W-cameleon | | | |
| without Ca | 688.47 | 321.5 | 275.6 |
| With Ca | 631.42 | 312.0 | 254.4 |
| Note: Figures in the table indicate the fluorescence intensities (arbitrary unit) obtained by measuring with PMT (photomultiplier tube). | | | |

INDUSTRIAL APPLICABILITY

According to the present invention, a change in the three-dimensional structure induced by a ligand can be monitored by FRET. The fluorescent indicator of the present invention can be produced *in situ* by introduction of a gene into a cell or a living organism. Thus, it is not necessary that a large amount of soluble recombinant protein be allowed to express and be purified, that the obtained protein be purified and labeled *in vitro,* and that it be then returned to a cell by microinjection. In addition, the fluorescent indicator of the present invention is able to target for a cell structure.

Moreover, when various fluorescent indicators using the conventional FRET are used in a method using fluorescent substances having different colors (spectra), they require a wide range of wavelength region for a single FRET observation. Thus, a simultaneous observation of other fluorescent dyes is limited. In contrast, since only a single color is basically used in the present invention, the wavelength region used for FRET observation can be narrowed, and thus, the present invention is suitable for imaging of multiple colors. For example, if FRET imaging is carried out by reduction in fluorescent anisotropy based on each of CFP, YFP, and RFP, three events can simultaneously be monitored in a single cell.

**Claims**

1.  A fluorescent indicator formed by binding fluorescent molecular components having substantially identical fluorescent properties to the N- and C-terminal sides of a target sequence, to which an analytical substance binds or reacts, so as to change the three-dimensional structure of the indicator.

2.  A fluorescent indicator, which comprises:

    a target sequence, to which an analytical substance binds or reacts, so as to change the three-dimensional structure of the indicator;
    a donor fluorescent molecular component that covalently fuses to the target sequence; and
    an acceptor fluorescent molecular component that covalently fuses to the target sequence,

    wherein the donor fluorescent molecule and the acceptor fluorescent molecule have substantially identical fluorescent properties, and wherein the three-dimensional structure of the target sequence is changed due to the analytical substance binding to the target sequence, and the relative positions or orientation of the donor and the acceptor molecular component are then changed, and it is highly likely that the polarization properties of fluorescence observed when such a fluorescent molecule is excited by irradiation light having certain polarization properties differ from those of the irradiation light (depolarization).

3. The fluorescent indicator according to claim 1 or 2, wherein the fluorescence molecular component is a fluorescent protein or a mutant thereof.

4. The fluorescent indicator according to any of claims 1 to 3, wherein the fluorescence molecular component is a yellow fluorescent protein or a mutant thereof.

5. The fluorescent indicator according to any of claims 1 to 4, wherein the fluorescence molecular component is a fluorescent protein Venus.

6. The fluorescent indicator according to any of claims 1 to 5, wherein the fluorescent indicator further comprises a target peptide component and a linker component, wherein the target sequence of the analytical substance further comprises a peptide-binding domain for allowing the target peptide component to bind thereto,
wherein the linker component allows the target sequence of the analytical substance to covalently fuse to the target peptide component, and the target sequence and the target peptide component covalently fuse to either the acceptor fluorescent molecular component or the donor fluorescent molecular component, and
wherein the analytical substance binding to the target sequence induces a change in the relative positions or directions of the target peptide component and the peptide-binding domain, and the relative positions or directions of the donor and acceptor molecular component are then changed, and it is thereby highly likely that the polarization properties of fluorescence observed when such a fluorescent molecule is excited by irradiation light having certain polarization properties differ from those of the irradiation light (depolarization).

7. The fluorescent indicator according to any of claims 1 to 6, wherein the target sequence is calmodulin, cGMP-dependent protein kinase, a steroid hormone receptor, a ligand-binding domain of a steroid hormone receptor, protein kinase C, inositol-1,4,5-triphosphate receptor, or recobelin.

8. The fluorescent indicator according to claim 7, wherein the target sequence of the analytic substance is calmodulin.

9. The fluorescent indicator according to claim 6, wherein the target peptide component is skeletal muscle myosin light chain kinase (skMLCKp), smooth muscle myosin light chain kinase (smMLCK), calmodulin kinase II (CaMKII), caldesmon, calspermine, phosphofructokinase, calcineurin, phosphorylase kinase, $Ca^{2+}$-ATPase, 59 Kda phosphodiesterase (PDE), 60 Kda phosphodiesterase (PDE), nitric oxide synthase, type I adenylyl cyclase, *Bordetella pertussis* adenylyl cyclase, neuromodulin, spectrin, myristoylated alanine-rich C kinase substrate (MARCKS), Mac-MARCKS(F52), b-Adducin, heat shock protein HSP90a, human immunodeficiency virus envelope glycoprotein 160 (HIV-1 gp160), brush-boarder myosin heavy chain-I (BBMHBI), dilute myosin heavy chain (MHC), mastoparan, melittin, glucagon, secretin, vasoactive intestinal peptide (VIP), gastrin inhibitory peptide (GIP), or a calmodulin-binding domain of calmodulin-binding peptide-2 (Model peptide CBP2).

10. The fluorescent indicator according to claim 6, wherein the linker component is a peptide component.

11. The fluorescent indicator according to claim 10, wherein the linker component consists of 1 to 30 amino acid residues.

12. The fluorescent indicator according to any of claims 1 to 5, wherein the target sequence, on which an analytical substance reacts, so as to change the three-dimensional structure of the indicator, is an amino acid sequence that is cleaved with enzymes.

13. The fluorescent indicator according to any of claims 1 to 12, which is a single polypeptide.

14. The fluorescent indicator according to any of claims 1 to 13, which further comprises a localized sequence.

15. The fluorescent indicator according to any of claims 1 to 14, wherein the localized sequence is a nucleus-localized sequence, an endoplasmic reticulum-localized sequence, a peroxisome-localized sequence, a mitochondrion-localized sequence, a Goldi apparatus-localized sequence, or a cell membrane-localized sequence.

16. A method for detecting or measuring an analytical substance in a sample, which comprises:

    (1) a step of allowing a sample to interact with the fluorescent indicator of any of claims 1 to 15;
    (2) a step of exciting a donor component; and
    (3) a step of measuring the level of fluorescence resonance energy transfer in the indicator that reflects the concentration or activity of the analytical substance in the sample.

17. The method according to claims 16 wherein the level of fluorescence resonance energy transfer in the indicator is measured by depolarization.

18. The method according to claims 17 wherein the depolarization is measured by obtaining fluorescence anisotropy.

19. The method according to any of claims 16 to 18 wherein the sample is a living cell, and the step comprises incorporation of the fluorescent indicator into the cell.

20. The method according to claims 19 wherein the step of incorporating the fluorescent indicator into a cell comprises transfection of the cell with an expression vector containing an expression regulatory sequence that is functionally ligated to a nucleic acid sequence encoding the expression of the fluorescent indicator.

21. A nucleic acid encoding the fluorescent indicator of any of claims 1 to 15.

22. An expression vector containing the nucleic acid of claim 21.

23. A transformant having the nucleic acid of claim 21 or the expression vector of claim 22.

Fig. 1

Venus —Asp—Glu—Val—Asp— Venus

Venus — calmodulin — M13 — Venus

## INTERNATIONAL SEARCH REPORT

| | |
|---|---|
| International application No. | |
| | PCT/JP03/15790 |

**A. CLASSIFICATION OF SUBJECT MATTER**
Int.Cl⁷ G01N33/68, 33/483, C12N15/09, 1/21, 5/10, C07K14/435

According to International Patent Classification (IPC) or to both national classification and IPC

**B. FIELDS SEARCHED**

Minimum documentation searched (classification system followed by classification symbols)
Int.Cl⁷ G01N33/68, 33/483, C12N15/09, 1/21, 5/10, C07K14/435

Documentation searched other than minimum documentation to the extent that such documents are included in the fields searched
| | | | |
|---|---|---|---|
| Jitsuyo Shinan Koho | 1922–1996 | Toroku Jitsuyo Shinan Koho | 1994–2004 |
| Kokai Jitsuyo Shinan Koho | 1971–2004 | Jitsuyo Shinan Toroku Koho | 1996–2004 |

Electronic data base consulted during the international search (name of data base and, where practicable, search terms used)
BIOSIS

**C. DOCUMENTS CONSIDERED TO BE RELEVANT**

| Category* | Citation of document, with indication, where appropriate, of the relevant passages | Relevant to claim No. |
|---|---|---|
| A | NATURE, Vol.388, (1997), pages 882 to 887 | 1-23 |
| A | JP 2002-153279 A (Chief of Okazaki National Research Institutes), 28 May, 2002 (28.05.02), & EP 1209167 A | 1-23 |
| A | WO 00/073437 A (MERCH FROSST CANADA & CO.), 07 December, 2000 (07.12.00), & JP 2003-501024 A | 1-23 |
| A | BIOPHYSICAL JOURNAL, Vol.80, (2001), pages 3000 to 3008 | 1-23 |

☐ Further documents are listed in the continuation of Box C. ☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier document but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| | |
|---|---|
| Date of the actual completion of the international search | Date of mailing of the international search report |
| 11 March, 2004 (11.03.04) | 23 March, 2004 (23.03.04) |
| Name and mailing address of the ISA/ | Authorized officer |
| Japanese Patent Office | |
| Facsimile No. | Telephone No. |

Form PCT/ISA/210 (second sheet) (July 1998)

SEQUENCE LISTING

<110> RIKEN

<120> Fluorescent indicator using FRET

<130> A31676A

<160> 45

<210> 1

<211> 26

<212> PRT

<213> animal

<400> 1

Lys Arg Arg Trp Lys Lys Asn Phe Ile Ala Val Ser Ala Ala Asn Arg
1               5                   10                  15

Phe Lys Lys Ile Ser Ser Ser Gly Ala Leu
            20                  25

<210> 2

<211> 20

<212> PRT

<213> animal

<400> 2

Ala Arg Arg Lys Trp Gln Lys Thr Gly His Ala Val Arg Ala Ile Gly
1               5                   10                  15

Arg Leu Ser Ser
            20

<210> 3

<211> 20

<212> PRT

<213> animal

<400> 3

Ala Arg Arg Lys Leu Lys Gly Ala Ile Leu Thr Thr Met Leu Ala Thr
1               5                   10                  15

Arg Asn Phe Ser
            20

<210> 4

<211> 17

<212> PRT

<213> animal

<400> 4

Gly Val Arg Asn Ile Lys Ser Met Trp Glu Lys Gly Asn Val Phe Ser
1               5                   10                  15

Ser

<210> 5

<211> 20

<212> PRT

<213> animal

<400> 5

Ala Arg Arg Lys Leu Lys Ala Ala Val Lys Ala Val Val Ala Ser Ser
1               5                   10                  15

Arg Leu Gly Ser
            20

<210> 6

<211> 26

<212> PRT

<213> animal

<400> 6

Phe Met Asn Asn Trp Glu Val Tyr Lys Leu Leu Ala His Ile Arg Pro
1 5 10 15

Pro Ala Pro Lys Ser Gly Ser Tyr Thr Val
20 25

<210> 7

<211> 24

<212> PRT

<213> animal

<400> 7

Ala Arg Lys Glu Val Ile Arg Asn Lys Ile Arg Ala Ile Gly Lys Met
1 5 10 15

Ala Arg Val Phe Ser Val Leu Arg
20

<210> 8

<211> 26

<212> PRT

<213> animal

<400> 8

Leu Arg Arg Leu Ile Asp Ala Tyr Ala Phe Arg Ile Tyr Gly His Trp
1 5 10 15

Val Lys Lys Gly Gln Gln Gln Asn Arg Gly
20 25

<210> 9

<211> 27

<212> PRT

<213> animal

<400> 9

Arg Gly Lys Phe Lys Val Ile Cys Leu Thr Val Leu Ala Ser Val Arg

1                5                   10                   15

Ile Tyr Tyr Gln Tyr Arg Arg Val Lys Pro Gly

20                   25

<210> 10

<211> 28

<212> PRT

<213> animal

<400> 10

Leu Arg Arg Gly Gln Ile Leu Trp Phe Arg Gly Leu Asn Arg Ile Gln

1                5                   10                   15

Thr Gln Ile Lys Val Val Asn Ala Phe Ser Ser Ser

20                   25

<210> 11

<211> 21

<212> PRT

<213> animal

<400> 11

Arg Arg Lys His Leu Gln Arg Pro Ile Phe Arg Leu Arg Cys Leu Val

1                5                   10                   15

Lys Gln Leu Glu Lys

20

<210> 12

<211> 21

<212> PRT

<213> animal

<400> 12

Thr Glu Lys Met Trp Gln Arg Leu Lys Gly Ile Leu Arg Cys Leu Val
1               5                   10                  15

Lys Gln Leu Glu Lys
                20

<210> 13

<211> 23

<212> PRT

<213> animal

<400> 13

Lys Arg Arg Ala Ile Gly Phe Lys Lys Leu Ala Glu Ala Val Lys Phe
1               5                   10                  15

Ser Ala Lys Leu Met Gly Gln
                20

<210> 14

<211> 28

<212> PRT

<213> animal

<400> 14

Ile Lys Pro Ala Lys Arg Met Lys Phe Lys Thr Val Cys Tyr Leu Leu
1               5                   10                  15

Val Gln Leu Met His Cys Arg Lys Met Phe Lys Ala
                20                  25

<210> 15

<211> 22

<212> PRT

<213> animal

<400> 15

Ala Cys Ile Asp Leu Leu Trp Lys Ile Ala Arg Ala Gly Ala Arg Ser
1               5               10              15

Ala Val Gly Thr Glu Ala
20

<210> 16

<211> 27

<212> PRT

<213> animal

<400> 16

Lys Ala His Lys Ala Ala Thr Lys Ile Gln Ala Ser Phe Arg Gly His
1               5               10              15

Ile Thr Arg Lys Lys Leu Lys Gly Glu Lys Lys
20              25

<210> 17

<211> 24

<212> PRT

<213> animal

<400> 17

Lys Thr Ala Ser Pro Trp Lys Ser Ala Arg Leu Met Val His Thr Val
1               5               10              15

Ala Thr Phe Asn Ser Ile Lys Glu
20

<210> 18

<211> 25

<212> PRT

<213> animal

<400> 18

Lys Lys Lys Lys Lys Arg Phe Ser Phe Lys Lys Ser Phe Lys Leu Ser

1        5        10        15

Gly Phe Ser Phe Lys Lys Ser Lys Lys

20        25

<210> 19

<211> 24

<212> PRT

<213> animal

<400> 19

Lys Lys Lys Lys Lys Phe Ser Phe Lys Lys Pro Phe Lys Leu Ser Gly

1        5        10        15

Leu Ser Phe Lys Arg Asn Arg Lys

20

<210> 20

<211> 31

<212> PRT

<213> animal

<400> 20

Lys Gln Gln Lys Glu Lys Thr Arg Trp Leu Asn Thr Pro Asn Thr Tyr

1        5        10        15

Leu Arg Val Asn Val Ala Asp Glu Val Gln Arg Asn Met Gly Ser

20        25        30

<210> 21

<211> 21

<212> PRT

<213> animal

<400> 21

Lys Asp Gln Val Ala Asn Ser Ala Phe Gln Glu Arg Leu Arg Lys His
1               5                   10                  15

Gly Leu Glu Val Ile
                20

<210> 22

<211> 21

<212> PRT

<213> animal

<400> 22

Tyr His Arg Leu Arg Asp Leu Leu Leu Ile Val Lys Arg Ile Val Glu
1               5                   10                  15

Leu Leu Gly Arg Arg
                20

<210> 23

<211> 23

<212> PRT

<213> animal

<400> 23

Gln Gln Leu Ala Thr Leu Ile Gln Lys Thr Tyr Arg Gly Trp Arg Cys
1               5                   10                  15

Arg Thr His Tyr Gln Leu Met
                20

<210> 24

<211> 24

<212> PRT

<213> animal

<400> 24

Arg Ala Ala Cys Ile Arg Ile Gln Lys Thr Ile Arg Gly Trp Leu Leu
1                 5                    10                   15

Arg Lys Arg Tyr Leu Cys Met Gln
                20

<210> 25

<211> 12

<212> PRT

<213> animal

<400> 25

Ile Asn Leu Lys Ala Ala Leu Ala Lys Lys Ile Leu
1                5                    10

<210> 26

<211> 26

<212> PRT

<213> animal

<400> 26

Gly Ile Gly Ala Val Leu Lys Val Leu Thr Thr Gly Leu Pro Ala Leu
1                 5                    10                   15

Ile Ser Trp Ile Lys Arg Lys Arg Gln Gln
                20                   25

<210> 27

<211> 30

<212> PRT

<213> animal

<400> 27

His Ser Gln Gly Thr Phe Thr Thr Ser Asp Tyr Ser Lys Tyr Leu Asp
1                5                    10                   15

Ser Arg Arg Ala Gln Asp Phe Val Gln Trp Leu Met Asn Thr
                    20                  25                  30

<210> 28

<211> 27

<212> PRT

<213> animal

<400> 28

His Ser Asp Gly Thr Phe Thr Ser Glu Leu Ser Arg Leu Arg Asp Ser
      1               5                   10                  15

Ala Arg Leu Gln Arg Leu Leu Gln Gly Leu Val
                    20                  25

<210> 29

<211> 28

<212> PRT

<213> animal

<400> 29

His Ser Asp Ala Val Phe Thr Asp Asn Tyr Thr Arg Leu Arg Lys Gln
      1               5                   10                  15

Met Ala Val Lys Lys Tyr Leu Asn Ser Ile Leu Asn
                    20                  25

<210> 30

<211> 33

<212> PRT

<213> animal

<400> 20

Tyr Ala Asp Gly Thr Phe Ile Ser Asp Tyr Ser Ala Ile Met Asn Lys
      1               5                   10                  15

Ile Arg Gln Gln Asp Phe Val Asn Trp Leu Leu Ala Gln Gln Gln Lys
                20                  25                  30

Ser

<210> 31

<211> 17

<212> PRT

<213> animal

<400> 31

Lys Leu Trp Lys Lys Leu Leu Lys Leu Leu Lys Lys Leu Leu Lys Leu
    1               5                   10                  15

Gly

<210> 32

<211> 5

<212> PRT

<213> eucaryotic cell

<400> 32

Lys Lys Lys Arg Lys
                    5

<210> 33

<211> 26

<212> PRT

<213> eucaryotic cell

<400> 33

Met Leu Arg Thr Ser Ser Leu Phe Thr Arg Arg Val Gln Pro Ser Leu
    1               5                   10                  15

Phe Arg Asn Ile Leu Arg Leu Gln Ser Thr
                20                  25

<210> 34

<211> 4

<212> PRT

<213> eucaryotic cell

<400> 34

Lys Asp Glu Leu

<210> 35

<211> 3

<212> PRT

<213> eucaryotic cell

<400> 35

Ser Lys Phe

<210> 36

<211> 4

<212> PRT

<213> eucaryotic cell

<220>

<221> unsure

<222> 4

<223> unknown

<400> 36

Cys Ala Ala Xaa

<210> 37

<211> 2

<212> PRT

<213> eucaryotic cell

<400> 37

Cys Cys

<210> 38

<211> 3

<212> PRT

<213> eucaryotic cell

<220>

<221> unsure

<222> 2

<223> unknown

<400> 38

Cys Xaa Cys

<210> 39

<211> 4

<212> PRT

<213> eucaryotic cell

<220>

<221> unsure

<222> 3

<223> unknown

<220>

<221> unsure

<222> 4

<223> unknown

<400> 39

Cys Cys Xaa Xaa

<210> 40

<211> 29

<212> DNA

<213> Artificial Sequence

<220>

<223> Description of Artificial Sequence: Synthetic DNA

<400> 40

attggatccc atggtgagca agggcgagg                29

<210> 41

<211> 28

<212> DNA

<213> Artificial Sequence

<220>

<223> Description of Artificial Sequence: Synthetic DNA

<400> 41

catgcatgcg ggcggcggtc acgaactc                 28

<210> 42

<211> 28

<212> DNA

<213> Artificial Sequence

<220>

<223> Description of Artificial Sequence: Synthetic DNA

<400> 42

gctggtacca tggtgagcaa gggcgagg                 28

<210> 43

<211> 33

<212> DNA

<213> Artificial Sequence

<220>

<223> Description of Artificial Sequence: Synthetic DNA

<400> 43

gcagaattct cacttgtaca gctcgtccat gcc                    33

<210> 44

<211> 29

<212> DNA

<213> Artificial Sequence

<220>

<223> Description of Artificial Sequence: Synthetic DNA

<400> 44

attggatccc atggtgagca agggcgagg                    29

<210> 45

<211> 45

<212> DNA

<213> Artificial Sequence

<220>

<223> Description of Artificial Sequence: Synthetic DNA

<400> 45

gctggtacca tcgacctcat cagtgatccc ggcggcggtc acgaa            45